# EUROPEAN PATENT APPLICATION

(11) **EP 1 673 988 A1**
(43) Date of publication of application: **28.06.2006**
(21) Application number: 05077689.7
(22) Date of filing: 24.11.2005
(51) Int. Cl.: A23L 1/302, A23L 1/30, A61K 31/4415, A61K 31/355, A61K 31/352

(54) **Micronutrient formulation for cognitive & mood benefits**

(30) Priority: 22.12.2004 EP 04258034
(71) Applicant: UNILEVER PLC, London EC4P 4BQ (GB); UNILEVER N.V., 3013 AL Rotterdam (NL)
(72) Inventor: Cassidy, Aedin Margaret Mary, Norwich NR4 7QF (GB); O'Connor, Aine Martina, Bedfordshire MK44 1LQ (GB); Roxborough, Heather Elaine, Bedfordshire MK44 1LQ (GB)
(74) Representative: Hugot, Alain

(57) **Abstract**

A food product or dietary supplement is provided comprising (i) preferably at least 5 mg in total, of one or more isoflavones, (ii) preferably at least 0.1 mg, vitamin B6, and optionally (iii) preferably at least 1 mg, vitamin E. The food product or dietary supplement can be used to promote a positive mood state and/or improve cognitive function.

## Description

### Field of the invention

The present invention relates to micronutrient formulations and their use in providing mood and/or cognitive benefits.

### Background to the invention

With increasing proportions of aged individuals in the populations of most, if not all, countries, there is considerable interest in studying changes in mood states and cognitive functions that occurs with age. Negative mood states, which may lead to depression, increase the risk of age-related illnesses such as dementia or reduced heart health. Similarly, deteriorating cognitive function in late life substantially increases the risk of dementia and other age-related conditions. These changes are therefore associated with a significant public health burden. A number of studies have, for example, identified positive correlations between childhood IQ, occupation, duration of education in early life, and cognitive function later in life. However these factors are difficult or impossible to manipulate. Consequently there is a need for identifying suitable routes for reducing age-related changes in mood and cognitive function such that clinical or nutritional interventions can be readily administered.

US-A-2003/190369 describes multivitamin dietary supplements. Particular supplements are described which contain from 9 to 180 mg of soy isoflavones, from 5 to 100 mg of vitamin B6 and from 40 to 800 IU of vitamin E, as well as from 40 µg to 800 µg of folate. The vitamin B6 component is said to be important for 'mental and physical health'.

EP-A-1 214 893 discloses a composition comprising 200 IU vitamin E, 200 µg folic acid, 7.5 mg vitamin B6 and 40 mg isoflavones. It includes a suggestion to use such a composition to prevent, improve or cure mental decline.

### Summary of the invention

We have now found that, in a clinical trial, a combination of micronutrients, namely isoflavones and vitamin E improves mood state and/or cognitive function.

The present invention utilises a composition in a form suitable for systemic administration to an individual (i.e. a human), the composition comprising (i) one or more isoflavones, and (ii) vitamin E.

Therefore, the present invention provides a method of promoting a positive mood state and/or improving cognitive function in an individual which method comprises administering to a human, a composition as defined above. In one embodiment, the human is a post-menopausal female.

The present invention may also be expressed as use of a composition comprising (i) one or more isoflavones, and (ii) vitamin E in the manufacture of a product for promoting a positive mood state and/or improving cognitive function.

Components (i) and (ii), and indeed any optional additional components of the composition may be administered in combined form comprising two or more of such components and/or may be administered sequentially in separate form. Sequential administration preferably means administration of the relevant components within a maximum period of 5 minutes, more preferably within a maximum period of 2 minutes.

Preferably the composition further comprises at least 50 µg folic acid or a salt thereof.

Preferably the composition is in a form suitable for oral administration, more preferably a food product, dietary supplement or as a medicament.

Preferably the total amount of isoflavones is at least 5 mg.

Preferably the total amount of vitamin E is at least 1 mg.

Preferably the composition further comprises vitamin B6, more preferably at least 0.1 mg vitamin B6.

Preferably the composition further comprises folic acid or a salt thereof, more preferably at least 50 µg folic acid or a salt thereof.

Preferably the total amount of isoflavone administered per day is less than 80 mg.

### Detailed description of the invention

Unless defined otherwise, all technical and scientific terms used herein have the same meaning as commonly understood by one of ordinary skill in the art (e.g. in medicine and biochemistry)

### Compositions and Product Forms

Compositions of the invention/for use in the methods of the invention comprise (i) one or more isoflavones and (ii) vitamin E.

The total amount of isoflavones present in the composition is preferably at least 1 mg, more preferably at least 5 mg, such as at least 8 or 10 mg. In one embodiment, the total amount of isoflavones is less than 40 mg.

Preferably the isoflavones in the compositions of the invention are selected from genistein, daidzein and glycitein and mixtures thereof (or as glucons, i.e. in glycosylated form, which are known as genistin, daidzin and glycitin, respectively). Preferably the composition comprises genistein (or genistin) and daidzein (or daidzin).

The isoflavones are preferably derived from natural sources, typically plant materials - including extracts and/or concentrates thereof. Preferred sources of the isoflavones are legumes, such as soy, chick peas and/or clover, and extracts and/or concentrates thereof, most preferably soy.

As used herein, "vitamin E" includes alpha-, beta-, gamma- and delta-tocopherol in any isomeric form thereof or any mixture thereof (including mixtures of isomeric forms of any of these) as well as derivatives thereof. Derivatives of vitamin E can be oil-soluble or water-soluble. Examples of oil-soluble vitamin E derivatives, including ester derivatised vitamin E, tocopheryl acetate, tocopheryl linoleate, tocopheryl linoleate/oleate, tocopheryl nicotinate, and tocopherol (vitamin E alcohol). Water soluble vitamin E derivatives include sodium vitamin E phosphate (VEP), lauryl imino dipropionic acid tocopheryl phosphate, tocopheryl glucoside, tocopheryl succinate, tocophersolan (tocopheryl polyethylene glycol 1000 succinate), tocophereth-5, 10, 12, 18, and 50 (polyethylene glycol (PEG) tocopheryl ethers). For the PEG vitamin E derivatives, increasing numbers represent increasing numbers of PEG molecules attached to the vitamin E. Thus, as the number increases, so does water solubility, with tocopereth-5 having the lowest water solubility and tocopereth-50 having the greatest solubility in water. Derivatives of vitamin E as referred to herein have at least 50% of the biological activity of alpha-tocopherol, for example, at least 50% of the antioxidant activity of alpha-tocopherol.

The total amount of vitamin E is preferably at least 1 mg, more preferably at least 2 or 5 mg.

In a preferred embodiment, the composition further comprises vitamin B6, more preferably at least 0.1 mg vitamin B6, such as at least 0.2 or 0.5 mg.

In another preferred embodiment, the composition further comprises folic acid or a salt thereof. Preferably the total amount of folic acid or a salt thereof is at least 10 or 20 *µ*g, more preferably at least 50 or 100 *µ*g.

Other optional ingredients include one or more of green tea polyphenols (e.g. from 10 to 1000 mg); essential fatty acids (e.g. from 50 to 1000 mg) such as gamma-linolenic acid; calcium (e.g. from 50 mg to 2.5 g); zinc (e.g. from 1 to 40 mg); vitamin A (e.g. from 50 to 3000 µg); vitamin C (e.g. from 10 to 1000 mg); vitamin B2 (e.g. from 0.1 to 10 mg); vitamin B3 (e.g. from 1 to 35 mg); and vitamin D (e.g. from 0.5 to 50 *µ*g).

Compositions of the invention can be combined with an acceptable carrier or diluent, such as a pharmaceutically acceptable diluent or carrier, to produce a pharmaceutical composition or dietary/nutritional supplement. Pharmaceutically acceptable diluents or carriers suitable for use in such compositions are well known in the art of pharmacy. The compositions of the invention typically contain from 0.002 to 10% by weight of total active, such as from 0.2 to 5% by weight of active, more preferably at least 1 wt% of active.

The composition may consist of solid dosage forms such as tablets, hard gelatin capsules, soft gelatin capsules, bulk powders, and microcapsules. Alternately, it may consist of a liquid dosage form such as an aqueous or non-aqueous solution, emulsion, or suspension.

Solid compositions for oral administration are preferred compositions of the invention. Solid compositions of the invention are preferably prepared in unit dosage form, such as in the form of tablets and capsules. Suitably tablets may be prepared by mixing the active combination with an inert diluent such as calcium phosphate in the presence of disintegrating agents, for example maize starch, and lubricating agents, for example magnesium stearate, and tableting the mixture by known methods. Such tablets may, if desired, be provided with enteric coatings by known methods, for example by the use of cellulose acetate phthalate. Similarly, capsules, for example hard or soft gelatin capsules, containing the active combination optionally in the form of beads with or without added excipients, may be prepared by conventional means and, if desired, provided with enteric coatings in a known manner. The tablets may be formulated in a manner known to those skilled in the art so as to give a controlled release of the compound of the present invention.

Controlled release forms of the compositions of the present invention include rapid release formulations such as soluble granules or melt filled fast release capsules, delayed release formulations such as tablets provided with enteric coatings, for example, of cellulose acetate phthalate and, in particular, sustained release formulations. Numerous types of sustained release formulations are known to those skilled in the art. Typically, the active combination may be encapsulated within a release retarding coating, for example, a copolymer of cellulose ether and acrylate, or may be bound to small particles such as, for example, ion exchange resin beads. Alternatively, the active combination may be incorporated into a matrix containing a release retarding agent such as a hydrophilic gum e.g. xanthan gum, a cellulose derivative e.g. hydroxypropyl methylcellulose, or a polysaccharide, wax or plastics material.

The active combination may be formulated into a solid dosage form in which the active ingredients are kept separate. For example, the dosage form may be a bilayer tablet in which the active ingredients are contained in different layers. The different layers can be formulated so as to provide the optimum release profile for each active.

Liquid fill compositions for example viscous liquid fills, liquid paste fills or thixotropic liquid fills are also suitable for oral administration. Melt filled compositions may be obtained by mixing the active combination with certain esters of natural vegetable oil fatty acids, for example, the Gelucire™ range available from Gattefosse to provide a variety of release rates. Suitably a melt-filled capsule comprises from 10 to 80% active and from 20 to 90% of a fatty acid ester excipient which comprises one or more polyol esters and triglycerides of natural vegetable oil fatty acids.

Preferably oral liquid compositions comprise from 0.2 to 10 wt% active together with from 1 to 50 wt% of a diluent, the remainder made up with sterile water. Optionally the composition may contain suspending agents, thickeners, cosolvents such as alcohol and/or preservatives. Suitable diluents include sweetening agents for example sorbitol, xylitol or sucrose. Suitable suspending agents or thickeners include cellulose gums, agar or natural gums, for example xanthan gum. Flavourings or other taste-masking agents known to those skilled in the art for example saccharin, acesulpham K or aspartame may be added.

The preferred mode of administration is orally.

The compositions of the invention may also be provided as a food product. As used herein, the term "food products" includes both food products as such and beverages. Suitable food products as such include spreads, dairy products (including milk and yoghurts), desserts, convenience foods/snacks, breakfast cereals and cereal bars, mayonnaises, dressings, sandwich fillings, ready-cook meals, bread and frozen confections such as ice creams, water ices and sorbets and yoghurt ice creams. Suitable beverages include tea, tea-flavoured drinks, coffee, soft drinks (e.g. carbonated squashes etc) and fruit juice.

The food products are supplemented with the active ingredients of the invention so that they contain higher amounts of the active ingredient(s) than they would normally contain.

The food compositions of the invention typically contain from 0.02 to 10% by weight of total actives such as from 0.05 to 5% by weight of total active.

### Uses

The compositions of the invention can be used to promote a positive mood state in an individual and/or to improve cognitive function, especially individuals over the age of 40 or 50, such as post-menopausal women.

Examples of promoting positive mood states include reducing tension-anxiety, reducing confusion-bewilderment, lowering anger-hostility and/or protecting against a rise in fatigue-inertia.

Examples of improving cognitive function include improving memory (eg verbal memory, short-term memory and long-term memory); improving executive function (eg planning and prioritising); improving abstract reasoning and problem solving; improving attention & concentration; improving visuospatial function; improving psychomotor behaviour; improving processing speed; improving sensation and perception; and/or improving intelligence.

It is preferred that the daily dose of isoflavones provided by the compositions is 80 mg or less, preferably 60 or 50 mg or less. Preferably the daily dose of isoflavones provided by the compositions is greater than 10 mg.

The present invention will now be described with reference to the following examples which are illustrative only and non-limiting.

### EXAMPLES

### Introduction

Previous work (unpublished) carried out by the applicant found that post-menopausal women experienced increased tension-anxiety, fatigue-inertia, depression-dejection and confusion-bewilderment compared with their younger counterparts. Further, changes in cognitive function to decrease with age, with post-menopausal women exhibiting greater decrements in spatial working memory compared to aged matched males.

### Example 1

A nutrient cocktail, designed to improve mood and cognitive performance, was formulated. Efficacy of this formulation was assessed in a single centre, parallel, double-blind, randomised, placebo-controlled trial in post-menopausal US women.

### MATERIALS & METHODS

### Study Participants

33 healthy, post-menopausal women were recruited for the study with age ranging from 45 to 69 yrs (mean 56.1±4.3 yrs) and having an average BMI 26±4.3 kg/m². Subjects were all healthy individuals as determined by screening serum biochemistry and examination of their medical history. Post-menopausal was defined as ≥50 and ≤70 years and ammenorrhea for at least 12 months or ≥45 and ≤50 years and ammenorrhea for at least 2 years. Urine concentrations of E3G, P3G, LH and FSH were assayed to ensure that all subjects were indeed post-menopausal.

Subjects were excluded if they were currently or had been on HRT/ERT medication over the previous year, if they had smoked in the past year, had abnormal screening serum chemistry or were unwilling to stop taking nutritional supplements.

Subjects were randomised to either test or control with 17 subjects assigned to test product and 16 to placebo.

### Study Design

The intervention study comprised of two phases:

### (a) 2 week wash-out phase

Subjects were asked to stop intake of any antioxidant or dietary supplements and to adhere to a dietary restriction protocol, which prohibited consumption of any soy containing foods. Furthermore, subjects were asked to consume 2 placebo bars per day over this 2 week period.

### (b) 10 week intervention phase

Subjects were then randomized in a parallel design into two groups to receive either the nutrient test bar or placebo food. Both placebo and test bars contained 120mg PABA (para-aminobenzoic acid), urinary excretion of which was used as a measure of compliance. Subjects were asked to continue adherence to dietary restrictions described above and to consume the provided test or control product twice daily (once with breakfast and once with evening meal) over the 10 week period.

### Study Foods

Study foods were provided as a low-calorie bar (serving size 29g providing on average 108 calories and 3.1 g fat). PABA was added as a compliance marker to all the bars.

Each functional bar contained:

| | |
|---|---|
| Soya isoflavones | 20mg |
| Green tea polyphenols | 100mg |
| Gamma-linolenic acid | 240mg |
| Carotenoids | 0.25mg* |
| Vitamin A | 300µg |
| Vitamin C | 60mg |
| Vitamin E | 7.5mg |
| Vitamin B₂ | 0.55mg |
| Vitamin B₃ | 7mg |
| Vitamin B₆ | 0.75mg |
| Vitamin D | 5µg |
| Folate | 200µg |
| Zinc | 7.5mg |
| Calcium | 600mg |
| PABA | 120mg |

| | |
|---|---|
| * added at a non-functional level | |

The placebo foods contained PABA but none of the functional ingredients.

### Sample collection

Fasting venous blood samples were collected at the start of the 10 week intervention phase and on the last week of the intervention period. Serum and EDTA plasma samples were then collected following centrifugation at 3000rpm for 10mins at 4°C and aliquots stored at -80°C prior to analysis. Twenty-four hour urine samples were also collected at a total of 6 different time points through the study to monitor PABA excretion.

### Mood and Cognitive Assessments

The cognitive battery consisted of 3 psychometric questionnaires, the Everyday Memory Questionnaire (EMQ), Profile of Mood States (POMS) and General Health Questionnaire (GHQ), and a computerised cognitive test to assess spatial working memory. Subjects were asked to complete the questionnaires and the CANTAB (Cambridge Automated Neuro-psychological Test Battery) Spatial Working Memory test both pre and post 10 week dietary intervention.

### Everyday Memory Questionnaire (EMQ)

This questionnaire aims to provide an inventory of memory-related behaviours. Subjects were presented with 35 everyday 'memory failures' (e.g. forgetting a route) and asked to rate them for severity. Severity was determined by the reported frequency of the event over the past three months (e.g. once a week).

Responses were scored in terms of the dimensions 'retrieval', 'task monitoring', 'conversational monitoring', 'spatial memory' and 'memory for activities'.

### Profile of Mood States (POMS)

This is a shortened version of the full POMS questionnaire. A list of 36 emotional descriptions were presented to the subject, which can be combined into 6 dimensions - anger-hostility, fatigue-inertia, confusion-bewilderment, tension-anxiety, depression-dejection and vigour-activity. Subjects were asked to complete the questionnaire with the instruction to reflect on how they were feeling at that particular time.

### General Health Questionnaire (GHQ)

The GHQ-28 has 28 questions, which can be combined into four dimensions - somatic symptoms, anxiety and insomnia, social dysfunction and severe depression. A total health score is obtained by summing these dimensions, with higher scores indicating poorer health. Subjects filled in the questionnaire with the instruction to reflect on feelings they have had over the past few weeks.

### Spatial Working Memory (SWM)

Spatial working memory was assessed by a computer-based test using a touch-sensitive screen. A number of measures were taken during the task. These were termed 'between errors', 'within errors', 'total between errors', 'total within errors', 'total errors' and 'strategy'. The 'strategy' score was the measure of the ability of an individual to devise a strategy to aid completion of the task.

### Nutrient Analysis

Where technically possible, the serum/plasma levels of the nutrients, which were present in the bars, were assayed to determine the biological impact of supplementation with the fortified food products. Serum levels of genistein and daidzein were measured by a fluorescence immunoassay established in-house on the AutoDelfia. Concentrations of serum retinol, α-tocopherol, lycopene, α-carotene, β-carotene, lutein, zeaxanthin and β-cryptoxanthine were measured by HPLC with diode array detection. Serum pyridoxal-5-phosphate (vitamin B₆) concentrations were quantified using an HPLC method from Chromsystems (Munich, Germany). Serum folate was measured by a competitive protein binding method using a commercially available kit.

### Statistical Analysis

Analysis of variance of the differences from baseline was carried out for each of the questionnaire or SWM variables with the baseline score and age included as covariates and both the 'Test-Placebo' effect and the single effects of 'Test' or 'Placebo' analysed.

Regression analysis was also carried out in the whole study population to establish the relationship between the serum nutrient data and the SWM or questionnaire outputs both pre and post intervention.

### RESULTS

### Compliance

33 subjects completed the study. Compliance was judged to be good as assessed by both subjective and objective measures. Briefly, subjects completed a food diary, in which labels attached to study bars were peeled off and stuck once consumed. The maximum number of bars reported to be missed was 14 during the 12 week (and therefore 168 bar) trial. This was corroborated by the urinary PABA results, which were used as an objective measure of compliance. PABA recovery was found to be greater that 85% at all time points and so was greater than the minimum recovery thought to indicate compliance.

### Micronutrient Analysis

Serum or plasma levels of genistein, daidzein, vitamin A, vitamin E, carotenoids, vitamin B₆ and folate were measured. Significant increases in circulating levels of genistein (p<0.0001), daidzein (p=0.03), vitamin E (p=0.05), vitamin B₆ (p=0.004) and folate (p=0.005) were noted in the test group following supplementation, while levels in the control group remained unchanged. However, no significant changes were noted in vitamin A or carotenoid levels in the test group following intervention compared to the control confirming that carotenoids were added at a non-functional level.

### Everyday Memory Questionnaire (EMQ)

### Memory for Activities

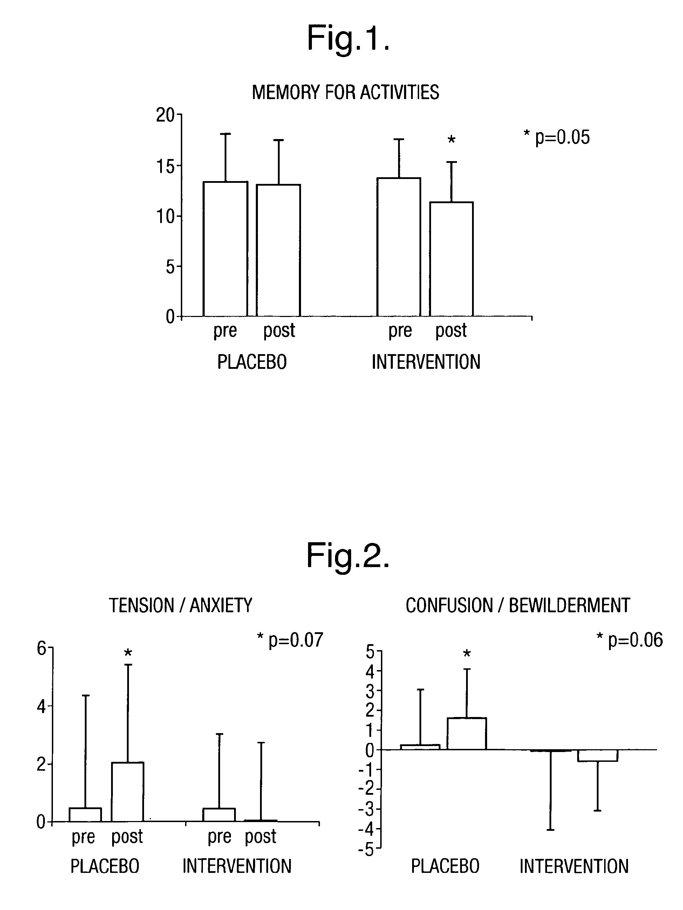

A significant improvement was noted following dietary intervention in the test group (p=0.002) but not in control. Furthermore, a significant difference was also observed when response in test group was compared to control (p=0.05) (see Figure 1).

### Profile of Mood States (POMS)

### Tension-Anxiety

No significant difference was observed in the test group pre and post intervention (0.5±2.53 to 0.1±2.66; p=0.57). However, a near-significant difference was observed in tension-anxiety between test and control group (p=0.067). This was largely due to the significant increase observed in the control group following intervention (0.5±3.85 to 2.1±3.34; p=0.04). Figure 2.

### Confusion-Bewilderment

Scores in the test group were found to decrease non-significantly post intervention with a near-significant difference observed between test and control (p=0.06). This was mainly due to the rise in confusion and bewilderment reported in the control group (0.3±2.77 to 1.7±4.03; p=0.068). Figure 2.

### Relationships between nutrient levels and mood & memory

Significant correlations were observed between change in two POMS dimensions (tension-anxiety and confusion-bewilderment) and serum isoflavone levels.

| | | |
|---|---|---|
| Tension-anxiety: | genistein | r -0.33, p=0.05 |
| | daidzein | r -0.37, p=0.03 |
| Confusion-bewilderment: | genistein | r -0.37, p=0.03 |
| | daidzein | r -0.47, p=0.005 |

### CONCLUSION

Supplementation with our nutrient formulation was shown to provide both mood and cognitive benefits in post-menopausal women.

### Mood

One of the most marked improvements noted following intervention was in mood states. Our formulation was found to protect against the rise in tension and anxiety and confusion and bewilderment shown to occur in the control group during the study.

### Memory

A distinct improvement which was noted following intervention was in the EMQ 'everyday memory for activities' factor which is the sum of subjects' responses to the following 5 questions - 'have you had to go back to check whether you have done something?', 'forgotten where you have put things?', 'forgotten a change in your daily routine?', 'forgotten to take things with you?', 'find television stories difficult to follow?'. Subjects consuming the nutrient bar reported a significant improvement in these aspects of memory compared to that of the placebo control.

### Key ingredients

Regression analysis revealed negative relationships between serum genistein and daidzein concentrations post-intervention and reports of confusion and bewilderment and tension and anxiety. It is, therefore, likely that soy isoflavones are one of the main ingredients responsible for the beneficial effects on mood observed. However, several nutrients present in the formulation were not measured and could also prove to be efficacious.

### Example 2

A nutrient cocktail, designed to improve mood was formulated. Efficacy of this formulation was assessed in a single centre, parallel, double-blind, randomised, placebo-controlled trial in post-menopausal US women.

### Subjects

The subjects were healthy, non-smoking, Caucasian, post-menopausal women, who were not on hormone replacement therapy (HRT) currently or in the past year. Post-menopausal was defined as ≥50 ≤65 years and ammenorrhea for at least 12 months or ≥45 ≤50 years and ammenorrhea for at least 2 years. Subjects had a BMI between 19 and 32 kg/m² and were screened to ensure they had 'normal American dietary habits'. 31 participants started the trial. However, three subjects were withdrawn during the trial due to abnormal serum biochemistry results. The remaining 28 completed the trial.

### Study Design

The intervention study comprised of two phases:

### (i) 2 week wash-out phase

Subjects were asked to consume placebo foods (4 portions per day) for 2 week period.

### (ii) 4 week intervention phase

Subjects were randomized in a parallel design into two groups to receive either the nutrient or placebo food (4 portions daily) for a 4 week period.

For the duration of the study the subjects had to avoid soya containing foods and stop taking any vitamins, minerals or other dietary supplements.

### Study Foods

Each test food portion contained the following ingredients:
10 mg of soya isoflavones (Novasoy 40, ADM)
3 mg of vitamin E
0.2 mg of vitamin B₆
0.6 µg of vitamin B₁₂
0.75 g of calcium
60mg of PABA (para-amino benzoic acid, a compliance marker).

The placebo foods contained PABA but none of the functional ingredients.

The subjects consumed 4 portions per day, one of each of the following food types:-
- Salad dressing: ranch or Italian flavour
- Soup: chicken noodle or tomato flavour
- Drink: hot chocolate (hot chai) or spiced apple cider
- Spread: strawberry

### Measurements

Blood sampling was performed after the run-in phase and at the end of the intervention. 24hr urine collections were taken weekly during the course of the study.

Mood and cognition were assessed using the General Health Questionnaire (GHQ) and Profile of Mood States (POMS) before and after dietary intervention period.

### RESULTS

### Compliance

28 subjects completed the study. Compliance was judged to be good as assessed by changes in biological concentrations of one of the test food ingredients - isoflavones.

### General Health Questionnaire

The General Health Questionnaire (GHQ) was completed by the subjects pre and post intervention. The questionnaire contained four sub-scales: severe depression, social dysfunction, somatic symptoms and sleep and anxiety, as well as a total health score.

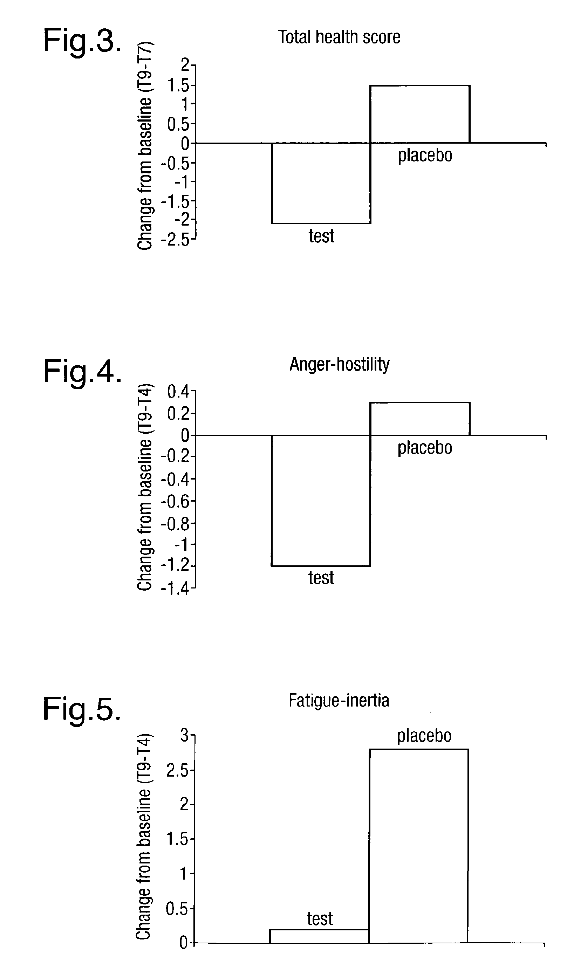

There were no significant changes in any of the sub-scales. However, significant improvements were noted in total health score in test group compared to control (p=0.05). Figure 3.

### Profile of Mood States

The Profile of Mood States (POMS) questionnaire was completed by the subjects pre and post intervention period. It covered five mood states: tense-arousal, depression-dejection, anger-hostility, fatigue-inertia and confusion-bewilderment.

There were significant treatment benefit effects for anger-hostility (p=0.052) with the test group reporting fewer feelings of 'anger' and 'grouchiness' than control group. Figure 4.

There was also a borderline significant treatment effect for fatigue-inertia (p=0.066). The test group showed no change in 'tiredness' or 'lethargy' while the placebo group reported an increase. This is suggestive of a protective effect, with the functional ingredients preventing the rise in fatigue-inertia reported by the control group during the study (see Figure 5).

### CONCLUSION

Supplementation with our nutrient formulation was shown to provide mood benefits in post-menopausal women.

The food products were found to improve total health score (as defined by GHQ), lower anger-hostility and protect against the rise in fatigue-inertia observed during the study.

The various features and embodiments of the present invention, referred to in individual sections above apply, as appropriate, to other sections, *mutatis mutandis*. Consequently features specified in one section may be combined with features specified in other sections, as appropriate.

All publications mentioned in the above specification are herein incorporated by reference. Various modifications and variations of the described methods and products of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in the relevant fields are intended to be within the scope of the following claims.

## Claims

1. Use of a composition comprising (i) one or more isoflavones, and (ii) vitamin E in the manufacture of a product for promoting a positive mood state and/or improving cognitive function.

2. Use according to claim 1 wherein the total amount of isoflavones is at least 5 mg.

3. Use according to claim 1 or claim 2 wherein the total amount of vitamin E is at least 1 mg.

4. Use according to any one of claims 1 to 3 wherein the composition further comprises vitamin B6.

5. Use according to claim 4 wherein the total amount of vitamin B6 is at least 0.1 mg.

6. Use according to any preceding claim, wherein the composition further comprises folic acid or a salt thereof.

7. Use according to claim 6 wherein the total amount of folic acid or a salt thereof is at least 50 µg.

8. Use according to any preceding claim, wherein the medicament is administered to a post-menopausal female.

9. Use according to any preceding claim, wherein the product is formulated as a food product.

10. Use according to any of claims 1 to 8, wherein the product is formulated as a dietary supplement.

11. Use according to any one of claims 1 to 8, wherein the product is formulated as a medicament.

12. Use according to any preceding claim, wherein the promotion of a positive mood state is selected from one or more of reducing tension-anxiety, reducing confusion-bewilderment, lowering anger-hostility and protecting against a rise in fatigue-inertia.

13. Use according to any preceding claim, wherein the improvement of cognitive function is selected from improving memory; improving executive function; improving abstract reasoning and problem solving; improving attention & concentration; improving visuospatial function; improving psychomotor behaviour; improving processing speed; improving sensation and perception; and improving intelligence.

14. A method of promoting a positive mood state and/or improving cognitive function in a human which method comprises administering to the individual a composition which comprises (i) one or more isoflavones and (ii) vitamin B6.

15. A method according to claim 14 wherein the patient is a post-menopausal female.
